# EUROPEAN PATENT SPECIFICATION

(11) **EP 3 179 946 B1**
(45) Date of publication and mention of the grant of the patent: **08.07.2020**
(21) Application number: 15832325.3
(22) Date of filing: 12.08.2015
(51) Int. Cl.: A61B 18/14, A61B 18/00, A61B 17/34, A61B 17/29

(54) **SURGICAL INSTRUMENT ELECTRODES**
ELEKTRODEN EINES CHIRURGISCHEN INSTRUMENTS
ÉLECTRODES D'INSTRUMENT CHIRURGICAL

(30) Priority: 13.08.2014 US 201462037073 P
(43) Date of publication of application: 21.06.2017
(73) Proprietor: Teleflex Medical Incorporated, Morrisville, North Carolina 27560 (US)
(72) Inventor: RAVIKUMAR, Sundaram, Briarcliff Manor, New York 10510 (US); ALWARD, Harry Allan, Shelton, Connecticut 06484 (US); OSBORNE, Guy, Trumbull, Connecticut 06611 (US)
(74) Representative: Nguyen-Van-Yen, Christian
(86) International application number: PCT/US2015/044760
(87) International publication number: WO 2016/025545

(56) References cited:
- EP-A1- 1 174 093
- US-A- 5 496 315
- US-A- 5 662 647
- US-A1- 2009 062 791
- US-A1- 2011 106 076
- US-A1- 2011 264 129
- US-B1- 6 740 079

## Description

### Technical Field

The present disclosure relates to surgical instruments and methods of their use, and more particularly to minimally invasive surgical instruments with electrodes and sealing means, and methods of use in surgery.

Examples of minimally invasive surgical assemblies and related equipment are described in U.S. Patent No. 7,766,937 to Ravikumar, U.S. Patent No. 8,230,863 to Ravikumar et al., U.S. Patent No. 8,313,507 to Ravikumar, U.S. Patent No. 8,133,255 to Ravikumar et al., U.S. Patent Application No. 11/685,522 to Ravikumar et al. (published as U.S. Patent Pub. No. 2007/0250112), U.S. Patent Application No. 12/503,035 to Ravikumar (published as U.S. Patent Pub. No. 2010/0016884), U.S. Patent Application No. 11/610,746 to Ravikumar et al. (published as U.S. Patent Pub. No. 2007/0282170), and U.S. Patent Application No. 12/689,352 to Ravikumar et al. (published as U.S. Patent Pub. No. 2010/0292724), as well as EP-A-1 174 093 and US-A-5 662 647.

### Description of Related Art

Over the last two decades, minimally invasive surgery has become the standard for many types of surgeries which were previously accomplished through open surgery. Minimally invasive surgery generally involves introducing an optical element (e.g., laparoscopic or endoscope) through a surgical or natural port in the body, advancing one or more surgical instruments through additional ports or through the endoscope, conducting the surgery with the surgical instruments, and withdrawing the instruments and scope from the body. In laparoscopic surgery (broadly defined herein to be any surgery where a port is made via a surgical incision, including but not limited to abdominal laparoscopy, arthroscopy, spinal laparoscopy, etc.), a port for a scope is typically made using a surgical trocar assembly.

The trocar assembly often includes a port, a sharp pointed element (trocar) extending through and beyond the distal end of the port, and at least in the case of abdominal laparoscopy, a sealing valve on the proximal portion of the port. The term trocar typically includes a combination of cooperating elements such as a cannula, a seal housing, and an obturator. First the obturator cuts or pierces the body wall so that the cannula may be inserted. The cannula defines a pathway through a body wall through which the surgical instruments are placed. Finally the seal housing provides an isolation of the cannula so that if insufflation is employed the body region remains distended and sealed. All three components are usually fitted together and used as a single unit for passage by one or more surgical instruments through the body wall and into a body cavity.

Laparoscopic surgery typically begins as the surgeon inserts a large bore needle through a body wall and into the internal region associated with the body wall. Next, an inflation or insufflation gas is pumped into the internal region until it is properly distended. The body wall and internal region are now ready for insertion of trocars.

If not already distended, an insufflation element may be attached to the trocar port in order to insufflate the surgical site. An optical element may then be introduced through the trocar port. Additional ports are then typically made so that additional laparoscopic instruments may be introduced into the body. Trocar assemblies are manufactured in different sizes. Typical trocar port sizes include diameters of about 5 mm, 10 mm, and 12 mm, which are sized to permit variously sized laparoscopic instruments to be introduced therethrough including, e.g., graspers, dissectors, staplers, scissors, suction/irrigators, clamps, forceps, biopsy forceps, etc. While 5 mm diameter trocar ports are relatively small, in some circumstances where internal working space is limited (e.g., children), it is difficult to place multiple 5 mm diameter ports in the limited area. In addition, 5 mm diameter trocar ports tend to limit movement of instruments inside the abdominal cavity to a great extent. Such a conventional 5 mm diameter trocar has a sealing valve and sealing mechanism and therefore the opening for the surgical instrument is limited. Thus, smaller diameter surgical access ports, such as those described in PCT/US2015/040371 entitled "Exchanger Surgical Access Port and Methods of Use" and PCT/US2014/056456 entitled "Minimally Invasive Surgical Re-Entry Exchanger Assembly and Methods" are useful in pediatric patients and in body locations where a smaller surgical access port is advantageous for surgery.

Further, while laparoscopic surgery has reduced the trauma associated with various surgical procedures and has concomitantly reduced recovery time from these surgeries, there always remains a desire in the art to further reduce the trauma to the patient.

One area of trauma associated with laparoscopic surgery identified by the inventors hereof as being susceptible of reduction are the scars which result from the trocar ports used. In many laparoscopic surgeries, three or more trocar incisions are made. For example, in laparoscopic hernia repair surgery, four trocar incisions are typically made, with one incision for insufflating the abdomen via a placed trocar and using such trocar for inserting the optical device, two incisions for placing trocar ports for inserting graspers therethrough, and a fourth port for passing a stapler therethrough. Those skilled in the art and those who have undergone surgical procedures understand that even the 5 mm diameter trocar ports leave holes which must be stitched and which result in scars. Scar tissue may affect the internal portion of the fascia as well as the cosmetic appearance of the skin, which may be detrimental for the patient or even a surgeon if that area of the skin is subject to a later incision or medical procedure. Thus a need exists for surgical methods which include fewer and smaller diameter trocars or surgical access ports.

Further, a need exists for a surgical instrument probe which has a smaller diameter to reduce trauma within the patient, even if used in connection with a trocar or surgical access port having a diameter of 5 mm or larger, thereby enabling use of smaller diameter instruments within the body cavity.

A further need exits for a surgical instrument probe which as a small diameter so as to reduce scarring at the surgical access location within the patient's body. A further need exists for a surgical instrument probe which has a small diameter to be used with a small diameter surgical access port.

A further need exits for a surgical instrument probe which as a small diameter and a longer length for use by a surgeon during surgery. Yet another need exists for a surgical instrument probe which has a smaller diameter, longer length, and end effectors such as a hook or spatula. A further need exists for a surgical instrument probe which has a smaller diameter, longer length with end effectors, such as a hook or spatula crimped to a rod within the surgical instrument probe.

Yet another need exists for a surgical instrument probe which has a smaller diameter, longer length, and a sealing means when used in connection with a large diameter trocar or surgical access port. A further need exists for a surgical instrument probe which has a smaller diameter and a sealing means to reduce or eliminate desufflation or gas leak when used in connection with a trocar or surgical access port having a larger diameter than that of the surgical instrument probe.

Other advantages of the present disclosure will become apparent from the following description and appended claims.

### Summary

According to the invention a surgical instrument probe according to claim 1 is provided. Preferred embodiments are defined in the dependent claims. The methods disclosed herein do not form part of the invention, but merely illustrates the intended use of the instrument probe of the invention. The surgical instrument probe includes an elongated shaft with a conductive rod, the elongated shaft defining an outer diameter of less than 3 mm and extending along an axial direction from a proximal end of the elongated shaft to a distal end of the elongated shaft. The surgical instrument probe includes a pencil handle hub attached to the elongated shaft on the proximal end of the elongated shaft, and includes an end effector connected to the distal end of the elongated shaft. The surgical instrument probe further includes a sealing means attached to the outer diameter of the elongated shaft.

### Brief Description of the Drawings

Figure 1 shows a perspective view of a surgical instrument probe with an L-shaped hook in accordance with aspects of the present disclosure.
Figure 2 shows a perspective view of a surgical instrument probe with a spatula end in accordance with aspects of the present disclosure.
Figure 3 shows a partial cross-sectional view of a distal end of a surgical instrument probe in accordance with aspects of the present disclosure.
Figure 4 shows a partial cross-sectional view of a proximal end of a surgical instrument probe in accordance with aspects of the present disclosure.
Figure 5 shows a partial cross-sectional view of a distal end of a surgical instrument probe in accordance with aspects of the present disclosure.
Figure 6 shows a partial cross-sectional view of the proximal end of the surgical instrument probe in accordance with aspects of the present disclosure.

### Detailed Description

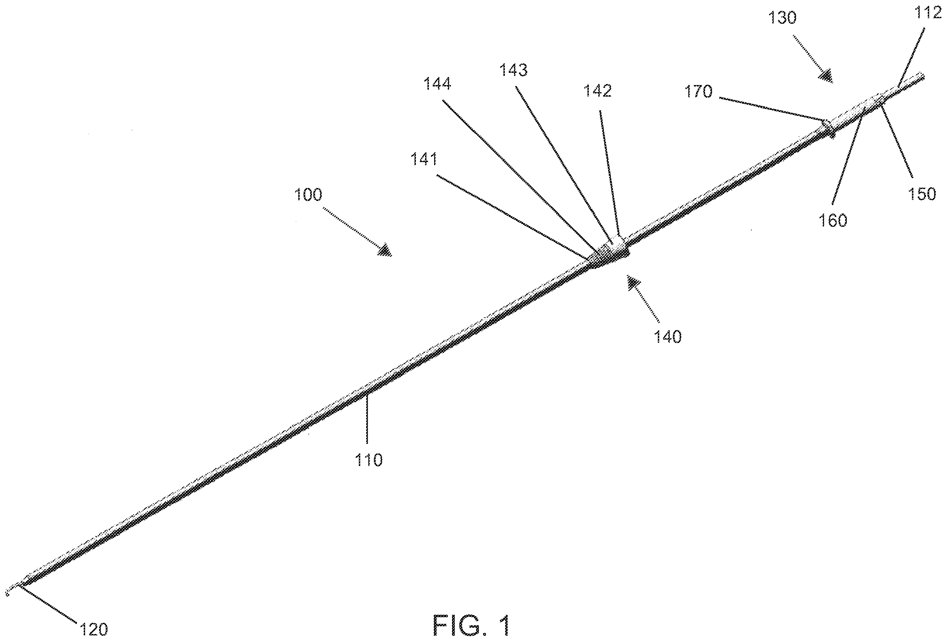

Reference will now be made to the drawings wherein like reference numerals identify similar structural features or aspects of the subject invention. For purposes of explanation and illustration, and not limitation, exemplary embodiments of a minimally invasive surgical assembly in accordance with the present disclosure, or aspects thereof, are shown in Figures 1 through 6. The surgical instrument probe of the present disclosure may provide a low cost, easy to manufacture, probe which can be used, for example, during minimally invasive surgical procedures to reduce trauma to a patient.

Figures 1 through 6 show a surgical instrument probe 100 which may include an elongated shaft 110 with a conductive rod 112 having an end effector 120, 125 on a distal end of the shaft 110, and a pencil handle hub 130 on a proximal end of the shaft 110. The surgical instrument probe 100 may also be referred to as an electrode when capable of being energized. The term "probe" and "electrode" may be used interchangeably.

The elongated shaft 110 of the surgical instrument probe 100 may have a diameter of less than about 3 mm (± 20%) and may be used in laparoscopic surgery so as to reduce scarring and complications for the patient. The surgical instrument probe 100 of the present disclosure may be manufactured with the end effector 120, 125 crimped to the conductive rod 112 within the shaft 110 for additional strength, continuity of conductivity if energized, and ease of manipulation of the surgical instrument probe 100 during use by the surgeon. The surgical instrument probe 100 of the present disclosure may be used in conjunction with a surgical access port having a diameter at the insertion point of less than about 3 mm.

Referring now to Figure 1, in one aspect, the surgical instrument probe 100 may include an elongated shaft 110, an end effector 120 (which may be a hook, such as an L-hook), and a pencil handle hub 130. The elongated shaft 110 may have a diameter of less than about 3 mm (± 20%), thereby reducing trauma to the patient and eliminating the need for a larger incision point through the fascia. The diameter of the elongated shaft 110 may preferably be less than about 3 mm, and more preferably between about 2.2 mm to about 2.95 mm. The surgical instrument probe 100 may have a length, measured from the proximal end to the distal end of the elongated shaft 110, of about 100 mm (for instance for use in pediatric applications) to about 400 mm (for instance when used in bariatric applications or in general use with obese patients). The surgical instrument probe 100 may have a length, measured from the proximal end to the distal end of the elongated shaft 110, of preferably about 200 mm to about 300 mm, and most preferably of about 250 mm. In one aspect, the conductive rod 112 may have a length of about 200 mm to about 300 mm, and more preferably the conductive rod 112 may have a length of about 250 mm. In one aspect, the conductive rod 112 may have diameter of less than about 3 mm.

The elongated shaft 110 includes a conductive rod 112 and may be electrically insulated on the outside with a plastic or other compatible material for insertion into a human cavity. If the surgical instrument probe 100 is used for electrocautery, the insulation may include certain dielectric properties.

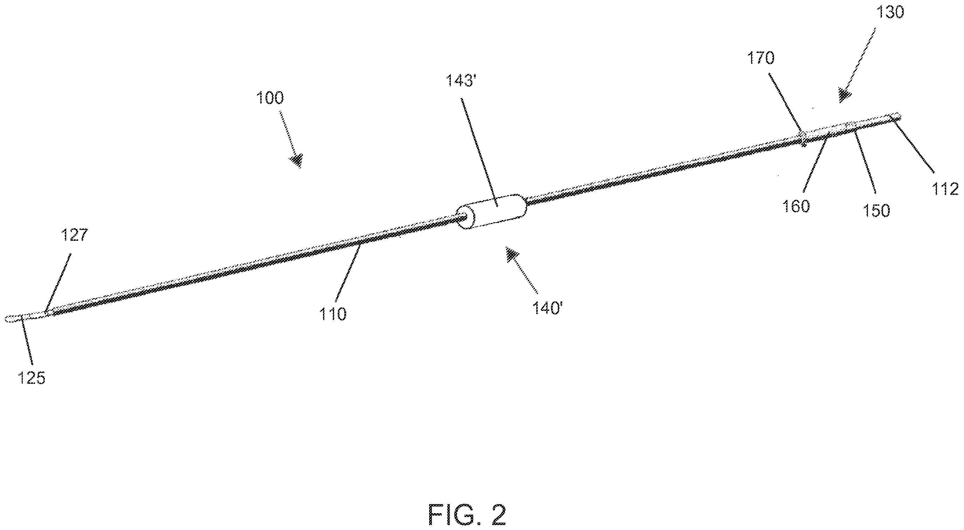

In one aspect, as shown in Figures 1 and 2, a sealing means 140, 140' may be attached to the outer diameter of the elongated shaft 110, and the outer diameter of the elongated shaft 110 may include insulation 114, as will be described in further detail below. The sealing means 140, 140' may be attached to the elongated shaft 110 between the end effector 120, 125, and a pencil handle hub 130. In one aspect, the sealing means 140 may comprise material that is soft and pliable such that it may be used as a seal between the surgical instrument probe 100 and an inner diameter of a surgical access port while enabling relative movement between the surgical instrument probe 100 and the trocar or surgical access port. The sealing means 140 may have elastic properties and may comprise materials such as rubber, silicon, polymers, and the like. In one aspect, the sealing means 140 may be compressible such that, when in use, it may be inserted between and expand to form a barrier or seal between the outer diameter of the surgical instrument probe 100 and the trocar or surgical access port. It is contemplated that the sealing means 140 may take on any shape necessary to provide a seal as described above. In one aspect, the outer diameter or shape of the sealing means 140 may mirror the inner diameter of the trocar or surgical access port.

Referring to Figure 1, the sealing means 140 may have a tapered conical outer surface 143 and may define an inner cannula for receiving the elongated shaft 110. The sealing means 140 may include a distal end 141 and a proximal end 142. In one aspect, an outer diameter of the distal end 141 may be less than an outer diameter of the proximal end 142. The larger diameter of the proximal end 142 of the sealing means 140 may help with insertion of the sealing member 140 into a trocar or surgical access portion and may provide a better seal or barrier with a top portion of the trocar or surgical access port.

In one aspect, the outer diameter of the distal end 141 may be less than or equal to an outer diameter of the elongated shaft 110. During installation and/or use, the elastic properties of the sealing means 140 may enable the inner cannula of the sealing means 140, at at least the distal end 141, to expand around the outer diameter of the elongated shaft 110, thereby providing a snug, sealing interface between the elongated shaft 110 and the sealing means 140.

In one aspect, the outer diameter of the proximal end 142 of the sealing means 140 may be greater than or equal to an inner diameter of the trocar or surgical access port. During use, the outer diameter of the proximal end 142 of the sealing means 140 may be compressed inwardly, for example in a radial direction, enabling the sealing means 140 to be at least partially inserted into the trocar or surgical access port. By at least partially compressing the proximal end 142 of the sealing means 140, a sealing interface may be provided between the sealing means 140 and the trocar or surgical access port. In one aspect, the inner diameter of the inner cannula of the sealing means 140 may be constant along an axial length of the sealing means 140 in order to provide a secure fit between the sealing means 140 and the elongated shaft 110, which may also include the insulation 114. In one aspect, the secure fit may be provided via friction between the contacting surfaces of the sealing means 140 and the elongated shaft 110. The one or more sealing interfaces of the sealing means 140 may eliminate or reduce desufflation or gas leak when the surgical instrument probe 100 is inserted into and used with the trocar or surgical access port.

In one aspect, the tapered conical outer surface 143 may include a bellows or ridges portion 145, and the ridges portion 145 may provide the surgical instrument probe 100 a greater range of reach and motion when inserted into the trocar or surgical access port with the sealing means 140 disposed therebetween. In one aspect, the ridges portion 145 may include a conical profile corresponding generally to a profile of the tapered conical outer surface 143 in the longitudinal axis direction of the sealing means 140. In one aspect, the ridges portion 145 may help promote sealing and retention with an inner diameter of the trocar or surgical access port during use. The retention function may be employed through the use of friction, and other grips and shapes may be employed on the sealing means 140 to provide sealing properties.

Referring to Figure 2, the sealing means 140' may have a cylindrical outer surface 143' and define an inner cannula for receiving the elongated shaft 110. The cylindrical outer surface 143' may have the same or substantially the same outer diameter along an axial length of the sealing means 140'. During installation and/or use, all of or portions of the cylindrical outer surface 143' may be compressed inwardly, for example in a radial direction, enabling the sealing means 140' to be at least partially inserted into the trocar or surgical access port. By at least partially compressing the cylindrical outer surface 143' against an inner surface of the trocar or surgical access port, a sealing interface may be provided to eliminate or reduce desufflation or gas leak when the surgical instrument probe 100 is inserted into and used with the trocar or surgical access port.

Although a tapered conical shape and a cylindrical shape has been described above for the sealing means 140, 140', respectively, other shapes are of course contemplated. For example, the sealing means may be in the shape of a cone, elliptical, circular, spherical, square, or any other known shape. The sealing means should have an opening through which the surgical instrument probe 100 may be inserted into.

The elongated shaft 110 may be connected on its distal end to an end effector 120. In Figure 1 the end effector 120 is a hook, shown as a L-hook shape. Other hook shapes are of course contemplated by the present disclosure and may include, without limitation, a J-hook, an eye-hook, or the like. In one aspect, other conventional end effectors may be implemented and connected during manufacture of the surgical instrument probe 100. In one aspect, the end effectors 120 may be attached and secured to the distal end of the elongated shaft 110 after the manufacture of the surgical instrument probe 100. For example, an end effector may be inserted into the distal end of the elongated shaft 110 and secured by means of at least one or more of a fastener and a threaded attachment. In one aspect, the end effector may be detachably removed from the elongated shaft 110 and replaced with another end effector.

The pencil handle hub 130 may be attached on the proximal end of the elongated shaft 110. The pencil handle hub 130 may include a flange 170, a hub surface 160, and an anti-rotational feature 150. In one aspect, the anti-rotation feature 150 may be configured to prevent relative rotation between the pencil handle hub 130 and the conductive rod 112. The anti-rotation feature 150 may include one or more of a friction material, a compression fitting, radially extending protrusions, and radially extending slots. The anti-rotation feature 150 may contact and/or interface with an outer surface of the conductive rod 112 to prevent the conductive rod 112 from rotating or shifting relative to the pencil handle hub 130.

The proximal end of the conductive rod 112 may extend out of the pencil handle hub 130, and the surgical instrument probe 100 may be configured for connection with an energy source for cauterization of the tissue adjacent to the end effector 120, 125. In use, the surgeon may insert the surgical instrument probe 100 into a conventional electrosurgical pencil (not shown) via the proximal end of the conductive rod 112, which extends out of the pencil handle hub 130. The surgeon has the ability to rotate the electrosurgical pencil without having the inserted surgical instrument probe 100 rotate during use due to an anti-rotational feature 150 of the pencil handle hub 130, thereby keeping the manipulation of the end effector 120, 125 in a constant and predictable location.

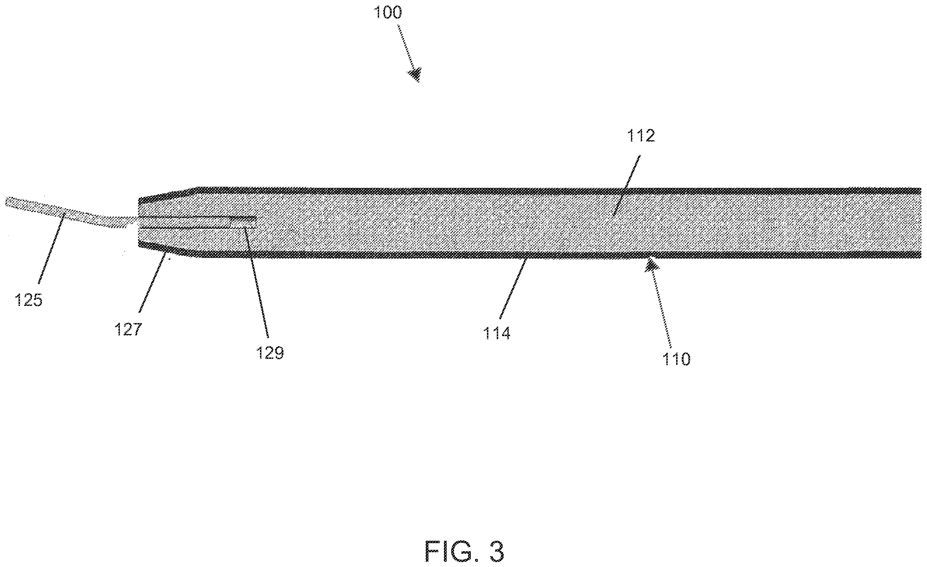

In one aspect, Figure 3 shows a partial cross-sectional view of the distal end of the surgical instrument probe 100 with the elongated shaft 110 wherein a diameter of the elongated shaft 110 is between about 2.2 mm to about 2.5 mm, and preferably between about 2.4 mm. The surgical instrument probe 100 may include a conductive rod 112 extending the length of the elongated shaft 110. The end effector 120, 125 may be connected to the conductive rod 112 via a connecting means 121, such as a crimp. The distal end of the conductive rod 112 may define an opening or aperture 129 located adjacent the crimp 127 so as to secure the end effector 120, 125 to the conductive rod 112. The opening or aperture 129 may provide an allowance for tolerances.

The elongated shaft 110 may include or may be covered with insulation 114, as shown in Figures 3 through 6, to provide electrical insulation. The insulation 114 may have a width of about 0.25 mm and may therefore add to the aggregate outer diameter of the elongated shaft 110. The insulation 114 may be a heat shrink insulation or may be co-molded with the elongated shaft 110. The insulation 114 should be compatible with the human body and may be made of biocompatible plastic, polymers, and the like. The conductive rod 112 may be made of any material which is conductive, such as metal and the like. In one aspect, the conductive rod 112 may be made of stainless steel. The end effector 120, 125 (and crimp 127 if present) should be compatible to the human body and may be made of any biocompatible material which is conductive, such as metal and the like. The end effector 120, 125 may be made of stainless steel.

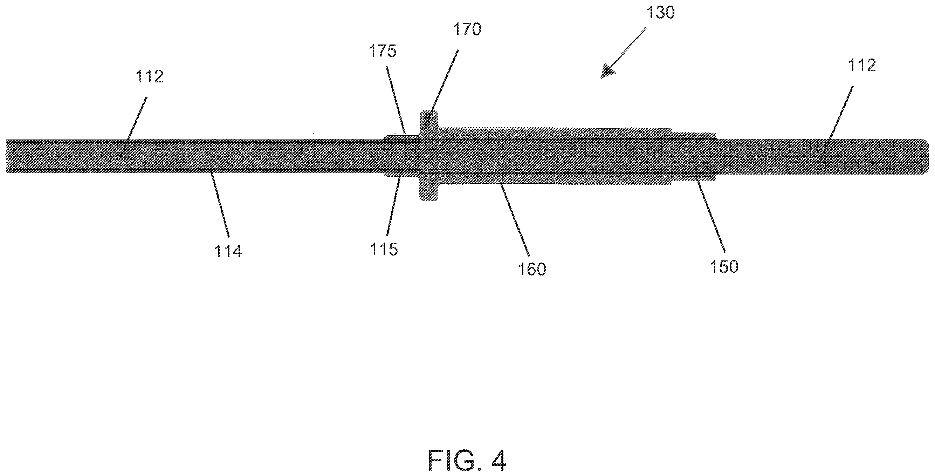

In one aspect, Figure 4 shows a partial cross-sectional view of the proximal end of the surgical instrument probe 100, the surgical instrument probe 100 including an elongated shaft 110 having an outer diameter of about 2.2 mm to about 2.6 mm, and preferably about 2.4 mm, inclusive of a thickness of the insulation 114. The surgical instrument probe 100 may include a conductive rod 112 with an overlay of insulation 114 and a proximal insulation end 115 may terminate at or within a distal portion 175 of the pencil handle hub 130. In one aspect, the proximal insulation end 115 extends along an inner diameter of the handle hub 130 at the distal portion 175. In one aspect, the interface between the proximal insulation end 115 and the distal portion 175 of the pencil handle hub 130 may serve as a sealing means to prevent any ingress or egress of fluids or gasses from passing through or between the conductive rod 112 and the pencil handle hub 130.

In one aspect, where the outer diameter of the elongated shaft 110 is about 2.4 mm, the proximal insulation end 115 extends along the inner diameter of the handle hub 130 and may extend up to a location the flange 170.

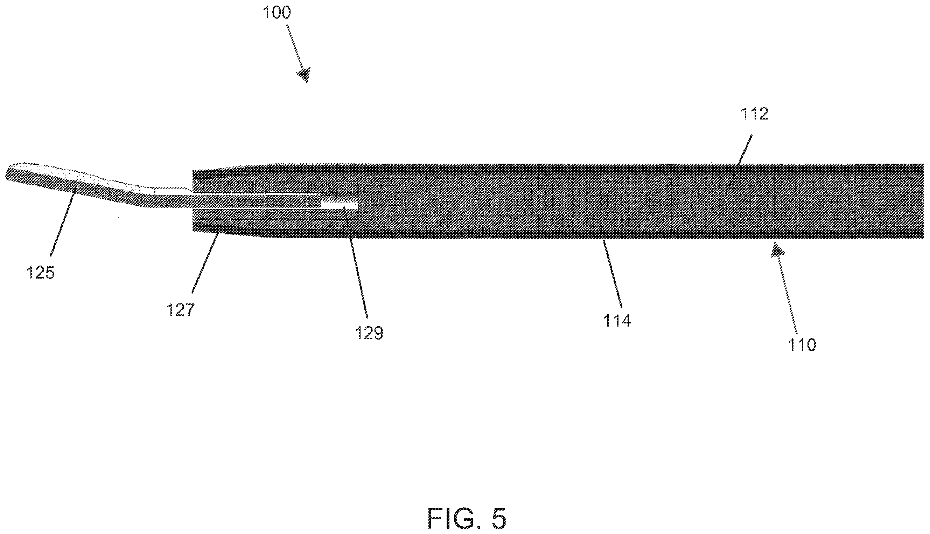

In one aspect, Figure 5 shows a partial cross-sectional view of the distal end of the surgical instrument probe 100, the surgical instrument probe 100 including an elongated shaft 110 having an outer diameter of about 2.6 mm to about 3.2 mm, and preferably about 2.9 mm. The surgical instrument probe 100 may include a conductive rod 112 extending at least a length of the elongated shaft 110. The conductive rod 112 may be connected to the end effector 120,125 via a connecting means 127, such as a crimp 127. The crimp 127 may define an opening or aperture 129 located adjacent to the end effector 125.

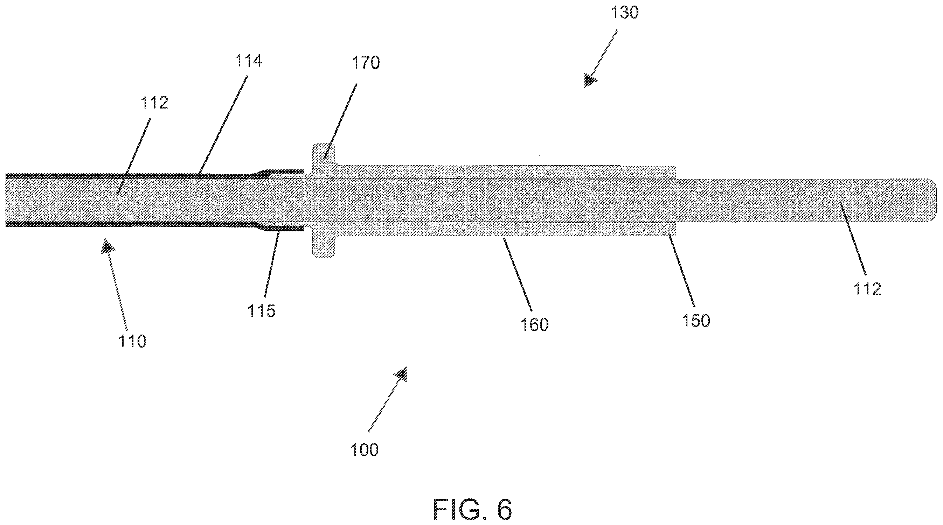

In one aspect, Figure 6 shows a partial cross-sectional view of the proximal end of the surgical instrument probe 100, the surgical instrument probe 100 having an elongated shaft 110 with an outer diameter of about 2.9 mm inclusive of the thickness of the insulation 114. The surgical instrument probe 100 may include a conductive rod 112 with an overlay of the insulation 114 and a proximal insulation end 115 may terminate and surround an outer diameter of the distal portion 175 of the pencil handle hub 130. In one aspect, a portion of the proximal insulation end 115 may surround the outer diameter of the distal portion 175 of the pencil handle hub 130 and may extend up to a location of the flange 170. In one aspect, the interface between the proximal insulation end 115 and the distal portion 175 of the pencil handle hub 130 may serve as a sealing means to prevent ingress or egress of any fluids or gasses from passing through or between the conductive rod 112 and the pencil handle hub 130.

In one aspect, the surgical instrument probe 100 may be an electrocautery and may be operable to cauterize target tissue positioned adjacent to the end effector 120, 125. In one aspect, the surgical instrument probe 100 may be connected to a monopolar or bipolar electrical means which may be used to cauterize the target tissue using the end effector 120, 125. In one aspect, the surgeon may use the end effector 120, 125 to hook and/or cut certain target tissue and then apply electrosurgical energy through the surgical instrument probe 100 to cauterize the target tissue. An electrosurgical treatment instrument may be provided with the surgical instrument probe 100 and may be capable of treating tissue via the use of heat, which may be produced using the electrosurgical energy, and the heat may be applied while contacting, cutting, and/or shearing the target tissue. The electrosurgical treatment instrument may be used to carryout operations or procedures, such as but not limited to, incisions, coagulations, and the like. During such a procedure, the electrosurgical treatment instrument may be equipped with an active electrode and an inactive, so-called neutral electrode. If the electrosurgical treatment instrument is monopolar, then during the whole duration of the surgery, the neutral electrode may be electrically connected to a large area of the patient's skin, which may include for example, the thigh or the upper arm of the patient.

In one aspect, the surgical instrument probe 100 may be inserted into an aperture of an electrosurgical pencil, and the surgical instrument probe 100 may be energized via a bipolar or monopolar means. The proximal end of the conductive rod 112 may extend beyond the proximal end of the pencil handle hub 130, which may be inserted into an aperture of the electrosurgical pencil, thereby allowing energy to run through the electrosurgical pencil, through the conductive rod 112 and crimp 127, and to the end effectors 120, 125. With the end effectors 120, 125 energized, target tissue in contact with, or in near contact with, the end effectors 120, 125 may be cauterized.

In one aspect, the electrosurgical treatment instrument or electrosurgical pencil may further comprise an electrical connector for connecting a conductor at the proximal end of the conductive rod 112 to an external electrosurgical generator. Electrical energy may be supplied to the surgical instrument by a conventional electrosurgical controls, which the user (e.g., surgeon) may activate via a button or switch, such as a foot switch, electrically connected to the electrosurgical generator. When the button or switch is triggered, the generator may supply electrical energy through a power cord to the electrical connector and onward to the surgical instrument probe 100. Typically a high frequency AC or RF current may be employed, and the voltage may be dependent on the type and degree of electrosurgical treatment desired. Voltages may range up to at least 12,000V in some cases, with about 3000V being a typical value used for coagulation, for example.

The surgical instrument probe 100 may be manufactured or produced by a process where the rod 200 is connected to the end effector 120, 125 via a connecting mean, such as the crimp 127, shown in Figures 3 and 5. The distal end of the conductive rod 112 may define a hole, opening, or aperture bored or formed within, which is capable of holding the proximal end of an end effector 120, 125. The distal end of the conductive rod 112, which contains the proximal end of the end effector 120, 125 is then connected via a connecting means. In one embodiment the connecting means is a crimping means wherein the distal end of the conductive rod 112 is compressed so as to retain or connect the conductive rod 112 with the proximal end of the end effector 120,125. Thus the end effector 120, 125 is connected and secured to the conductive rod 112. Either before or after the end effector 120, 125 is connected, the insulation 114 be added to the outer surface of the conductive rod 112. The insulation 114 protects the patient and any tissue or organs in contact with the outer diameter of the elongated shaft 110 during surgery while still allowing conductivity for the distal end of the end effector 120, 125 so as to cauterize or coagulate tissue in contact with the end effector 120, 125.

The surgical instrument probe may be used in surgery by inserting it into the patient's body cavity through various means, including direct insertion into the fascia which has already been cut, or through a trocar or other surgical access port such as that disclosed in PCT/US2015/040371 entitled "Exchanger Surgical Access Port and Methods of Use" and PCT/US2014/056456 entitled "Minimally Invasive Surgical Re-Entry Exchanger Assembly and Methods." The surgical instrument probe may be sold as a kit with the surgical access port and thus a less than about 3 mm diameter laparoscopic surgical kit may be packaged together.

The surgical instrument probe has the advantage of being small with a diameter or approximately 3 mm or less, preferably between about 2.2 mm to about 2.95 mm. The smaller diameter thus reduces the trauma to the patient with smaller surgical access port diameter and possibly less incisions in aggregate during the surgery.

The following benefits, structure, and advantages are also contemplated by the present invention: improved surgical precision, reduced surgical time resulting in reduced trauma to the patient and possibly less scarring, reduced recovery time, less pain, easier handling of the surgical instrument probe via the elongated shaft, and other benefits.

The surgical instrument probe is produced by starting with a rod, boring a hole within the distal end of the rod, inserting the proximal end of an end effector into the hole and joining the end effector with the rod via a connecting means, such as via crimping. In one aspect, the connecting means may be a crimping means and may include a compression means that forms a crimp 127, as shown in Figures 3 and 5. This crimp 127 is advantageous because it maintains continuity for end effector conductivity. In one aspect, the connecting means may be a heating means such as welding.

The surgical instrument probe 100 may be used in a variety of laparoscopic procedures. The methods and systems of the present disclosure, as described above and shown in the drawings, provide electrosurgical assemblies with small diameters to reduce scarring and improve maneuverability. While the surgical instrument electrodes and methods of the present disclosure have been shown and described, it will be appreciated that the foregoing description provides examples of surgical instrument probes which may be used with a surgical instrument for minimally invasive surgery.

However, it is contemplated that other implementations of the disclosure may differ in detail from the foregoing examples. All references to the disclosure or examples thereof are intended to reference the particular example being discussed at that point and are not intended to imply any limitation as to the scope of the disclosure more generally. All language of distinction and disparagement with respect to certain features is intended to indicate a lack of preference for those features, but not to exclude such from the scope of the disclosure entirely unless otherwise indicated.

Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

## Claims

1. A surgical instrument probe, comprising an elongated shaft (110), a pencil handle hub (130), an end effector (120, 125), and a sealing means (140, 140'), wherein:
the elongated shaft (110) includes a conductive rod (112), the elongated shaft (110) defining an outer diameter of less than 3 mm and extending along an axial direction from a proximal end of the elongated shaft (110) to a distal end of the elongated shaft (110);
the pencil handle hub (130) is attached to the elongated shaft (110) on the proximal end of the elongated shaft (110);
the end effector (120, 125) is connected to and at least partially inserted into an opening or aperture at the distal end of the elongated shaft (110); and
the sealing means (140, 140') is attached to the outer diameter of the elongated shaft (110), longitudinally between the pencil handle hub (130) and the end effector (120, 125), and the sealing means (140, 140') is configured to seal against a trocar or a surgical access port,
wherein the elongated shaft (110) includes insulation (114) extending along an outer surface of at least a length of the elongated shaft (110), and
wherein the insulation (114) extends from the distal end of the elongated shaft (110) to at least a distal portion of the pencil handle hub (130).

2. The surgical instrument probe of Claim 1, wherein the sealing means (140, 140') has elastic properties and is made of one or more of rubber, silicon, and polymers.

3. The surgical instrument probe of Claim 1, wherein the sealing means (140, 140') defines an inner cannula, and the inner cannula is configured to receive the elongated shaft (110).

4. The surgical instrument probe of Claim 3, wherein an inner diameter of the inner cannula is less than the outer diameter of the elongated shaft (110) prior to the sealing means being attached to the outer diameter of the elongated shaft (110).

5. The surgical instrument probe of Claim 1, wherein the sealing means (140, 140') includes a tapered conical outer surface or a cylindrical outer surface.

6. The surgical instrument probe of Claim 5, wherein the sealing means (140, 140') has the tapered conical outer surface (143), the sealing means (140, 140') including a distal end and a proximal end,
wherein the distal end of sealing means (140, 140') has a first outer diameter,
wherein the proximal end of the sealing means (140, 140') has a second outer diameter, and
wherein the first outer diameter is less that the second outer diameter, and
wherein the tapered conical outer surface (143) includes a bellows or ridges portion (145).

7. The surgical instrument probe of Claim 1, wherein the end effector (120, 125) is a spatula, a J-hook, or a L-hook.

8. The surgical instrument probe of Claim 1, wherein the pencil handle hub (130) defines an interior lumen extending from a proximal end of the pencil handle hub (130) to a distal end of the pencil handle hub (130).

9. The surgical instrument probe of Claim 8, wherein the interior lumen of the pencil handle hub (130) is attached to an outer surface of the elongated shaft (110) at the proximal end of the elongated shaft (110).

10. The surgical instrument probe of Claim 8, wherein the pencil handle hub (130) includes a flange (170) extending radially from an outer cylindrical surface of the pencil handle hub (130).

11. The surgical instrument probe of Claim 8, wherein the pencil handle hub (130) includes an anti-rotation feature (150) configured to prevent relative rotation between the pencil handle hub (130) and the elongated shaft (110), and
Wherein the end effector (120, 125) is secured within the opening or aperture via crimping.

12. The surgical instrument probe of Claim 1, wherein the elongated shaft (110) defines an opening or aperture at the distal end of the elongated shaft (110), and
wherein the end effector (120, 125) is at least partially inserted into the opening or aperture at the distal end of the elongated shaft (110), and the end effector (120, 125) is secured within the opening or aperture via crimping.

13. The surgical instrument probe of Claim 1, wherein the insulation (114) is heat shrink insulation.

14. The surgical instrument probe of Claim 1, wherein the insulation (114) extends over an outer surface of the distal portion of the pencil handle hub (130).

## Patentansprüche

1. Chirurgische Instrumentensonde, die einen länglichen Schaft (110), ein Stiftgriff-Verbindungsstück (130), einen Endeffektor (120, 125) und ein Abdichtungsmittel (140, 140') umfasst, wobei:
der längliche Schaft (110) einen leitfähigen Stab (112) einschließt, wobei der längliche Schaft (110) einen Außendurchmesser von weniger als 3 mm definiert und sich entlang einer axialen Richtung von einem proximalen Ende des länglichen Schafts (110) bis zu einem distalen Ende des länglichen Schafts (110) erstreckt,
das Stiftgriff-Verbindungsstück (130) an dem proximalen Ende des länglichen Schafts (110) an dem länglichen Schaft (110) befestigt ist,
der Endeffektor (120, 125) an dem distalen Ende des länglichen Schafts (110) mit einer Öffnung oder einem Loch verbunden und wenigstens teilweise in demselben eingesetzt ist, und
das Abdichtungsmittel (140, 140') an dem Außendurchmesser des länglichen Schafts (110), in Längsrichtung zwischen dem Stiftgriff-Verbindungsstück (130) und dem Endeffektor (120, 125), befestigt ist und das Abdichtungsmittel (140, 140') dafür konfiguriert ist, gegenüber einem Trokar oder einem chirurgischen Zugangsanschluss abzudichten,
wobei der längliche Schaft (110) eine Isolation (114) einschließt, die sich entlang einer Außenfläche wenigstens einer Länge des länglichen Schafts (110) erstreckt, und
wobei sich die Isolation (114) von dem distalen Ende des länglichen Schafts (110) bis zu wenigstens einem distalen Abschnitt des Stiftgriff-Verbindungsstücks (130) erstreckt.

2. Chirurgische Instrumentensonde nach Anspruch 1, wobei das Abdichtungsmittel (140, 140') elastische Eigenschaften aufweist und aus einem oder mehreren von Gummi, Silikon und Polymeren hergestellt ist.

3. Chirurgische Instrumentensonde nach Anspruch 1, wobei das Abdichtungsmittel (140, 140') eine innere Kanüle definiert und die innere Kanüle dafür konfiguriert ist, den länglichen Schaft (110) aufzunehmen.

4. Chirurgische Instrumentensonde nach Anspruch 3, wobei ein Innendurchmesser der inneren Kanüle geringer ist als der Außendurchmesser des länglichen Schafts (110), bevor das Abdichtungsmittel an dem Außendurchmesser des länglichen Schafts (110) befestigt wird.

5. Chirurgische Instrumentensonde nach Anspruch 1, wobei das Abdichtungsmittel (140, 140') eine verjüngte kegelförmige Außenfläche oder eine zylindrische Außenfläche einschließt.

6. Chirurgische Instrumentensonde nach Anspruch 5, wobei das Abdichtungsmittel (140, 140') die verjüngte kegelförmige Außenfläche (143) aufweist, wobei das Abdichtungsmittel (140, 140') ein distales Ende und ein proximales Ende einschließt,
wobei das distale Ende des Abdichtungsmittels (140, 140') einen ersten Außendurchmesser aufweist,
wobei das proximale Ende des Abdichtungsmittels (140, 140') einen zweiten Außendurchmesser aufweist und
wobei der erste Außendurchmesser geringer ist als der zweite Außendurchmesser und
wobei die verjüngte kegelförmige Außenfläche (143) einen Balgen- oder Stegabschnitt (145) einschließt.

7. Chirurgische Instrumentensonde nach Anspruch 1, wobei der Endeffektor (120, 125) ein Spatel, ein J-Haken oder ein L-Haken ist.

8. Chirurgische Instrumentensonde nach Anspruch 1, wobei das Stiftgriff-Verbindungsstück (130) ein inneres Lumen definiert, das sich von einem proximalen Ende des Stiftgriff-Verbindungsstücks (130) bis zu einem distalen Ende des Stiftgriff-Verbindungsstücks (130) erstreckt.

9. Chirurgische Instrumentensonde nach Anspruch 8, wobei das innere Lumen des Stiftgriff-Verbindungsstücks (130) an dem proximalen Ende des länglichen Schafts (110) an einer Außenfläche des länglichen Schafts (110) befestigt ist.

10. Chirurgische Instrumentensonde nach Anspruch 8, wobei das Stiftgriff-Verbindungsstück (130) einen Flansch (170) einschließt, der sich in Radialrichtung von einer äußeren zylindrischen Fläche des Stiftgriff-Verbindungsstücks (130) aus erstreckt.

11. Chirurgische Instrumentensonde nach Anspruch 8, wobei das Stiftgriff-Verbindungsstück (130) ein Drehsicherungsmerkmal (150) einschließt, das dafür konfiguriert ist, eine relative Drehung zwischen dem Stiftgriff-Verbindungsstück (130) und dem länglichen Schaft (110) zu verhindern, und
wobei der Endeffektor (120, 125) über Quetschen innerhalb der Öffnung oder des Lochs befestigt ist.

12. Chirurgische Instrumentensonde nach Anspruch 1, wobei der längliche Schaft (110) eine Öffnung oder ein Loch an dem distalen Ende des länglichen Schafts (110) definiert und
wobei der Endeffektor (120, 125) wenigstens teilweise in die Öffnung oder das Loch an dem distalen Ende des länglichen Schafts (110) eingesetzt ist und der Endeffektor (120, 125) über Quetschen innerhalb der Öffnung oder des Lochs befestigt ist.

13. Chirurgische Instrumentensonde nach Anspruch 1, wobei die Isolation (114) eine Wärmeschrumpfisolation ist.

14. Chirurgische Instrumentensonde nach Anspruch 1, wobei sich die Isolation (114) über eine Außenfläche des distalen Abschnitts des Stiftgriff-Verbindungsstücks (130) erstreckt.

## Revendications

1. Sonde d'instrument chirurgical, comprenant un tige allongée (110), un moyeu de poignée de crayon (130), un effecteur final (120, 125) et un moyen d'étanchéité (140, 140'), dans laquelle :
la tige allongée (110) inclut une tige conductrice (112), la tige allongée (110) définissant un diamètre extérieur inférieur à 3 mm et s'étendant le long d'une direction axiale d'une extrémité proximale de la tige allongée (110) vers une extrémité distale de la tige allongée (110) ;
le moyeu de poignée de crayon (130) est fixé sur la tige allongée (110) sur l'extrémité proximale de la tige allongée (110) ;
l'effecteur terminal (120, 125) est connecté à une ouverture ou à un orifice au niveau de l'extrémité distale de la tige allongée (110) est ou au moins partiellement insérée dans celle-ci ; et
le moyen d'étanchéité (140, 140') est fixé sur le diamètre extérieur de la tige allongée (110), de manière longitudinale entre le moyeu de poignée de crayon (130) et l'effecteur terminal (120, 125), et le moyen d'étanchéité (140, 140') étant configuré pour établir l'étanchéité par rapport à un trocart ou un orifice d'accès chirurgical ;
dans laquelle la tige allongée (110) inclut une isolation (114) s'étendant le long d'une surface externe d'au moins une longueur de la tige allongée (110) ; et
dans lequel l'isolation (114) s'étend de l'extrémité distale de la tige allongée (110) vers au moins une partie distale du moyeu de poignée de crayon (130).

2. Sonde d'instrument chirurgical selon la revendication 1, dans laquelle le moyen d'étanchéité (140, 140') présente des propriétés élastiques et est composé d'un ou de plusieurs parmi du caoutchouc, du silicone ou des polymères.

3. Sonde d'instrument chirurgical selon la revendication 1, dans laquelle le moyen d'étanchéité (140, 140') définit une canule interne, et la canule interne étant configurée pour recevoir la tige allongée (110).

4. Sonde d'instrument chirurgical selon la revendication 3, dans laquelle un diamètre intérieur de la canule interne est inférieur au diamètre extérieur de la tige allongée (110) avant la fixation du moyen d'étanchéité sur le diamètre extérieur de la tige allongée (110).

5. Sonde d'instrument chirurgical selon la revendication 1, dans laquelle le moyen d'étanchéité (140, 140') inclut une surface externe conique effilée ou une surface externe cylindrique.

6. Sonde d'instrument chirurgical selon la revendication 5, dans laquelle le moyen d'étanchéité (140, 140') comporte la surface externe conique effilée (143) le moyen d'étanchéité (140, 140') incluant une extrémité distale et une extrémité proximale ;
dans laquelle l'extrémité distale du moyen d'étanchéité (140, 140') a un premier diamètre extérieur ;
dans laquelle l'extrémité proximale du moyen d'étanchéité (140, 140') a un deuxième diamètre extérieur ; et
dans laquelle le premier diamètre extérieur est inférieur au deuxième diamètre extérieur ; et
dans laquelle la surface externe conique effilée (143) inclut une partie à soufflet ou à nervures (145).

7. Sonde d'instrument chirurgical selon la revendication 1, dans laquelle l'effecteur terminal (120, 125) est une spatule, un crochet en J ou un crochet en L.

8. Sonde d'instrument chirurgical selon la revendication 1, dans laquelle le moyeu de poignée de crayon (130) définit une lumière interne s'étendant d'une extrémité proximale du moyeu de poignée de crayon (130) vers une extrémité distale du moyeu de poignée de crayon (130).

9. Sonde d'instrument chirurgical selon la revendication 8, dans laquelle la lumière interne du moyeu de poignée de crayon (130) est fixée sur une surface externe de la tige allongée (110) au niveau de l'extrémité proximale de la tige allongée (110).

10. Sonde d'instrument chirurgical selon la revendication 8, dans laquelle le moyeu de poignée de crayon (130) inclut une bride (170) s'étendant radialement à partir d'une surface cylindrique externe du moyeu de poignée de crayon (130).

11. Sonde d'instrument chirurgical selon la revendication 8, dans laquelle le moyeu de poignée de crayon (130) inclut une structure anti-rotation (150) configurée pour empêcher une rotation relative entre le moyeu de poignée de crayon (130) et la tige allongée (110) ; et
dans laquelle l'effecteur terminal (120, 125) est fixé dans une ouverture ou un orifice par sertissage.

12. Sonde d'instrument chirurgical selon la revendication 1, dans laquelle la tige allongée (110) définit une ouverture ou un orifice au niveau de l'extrémité distale de la tige allongée (110) ; et
dans laquelle l'effecteur terminal (120, 125) est au moins partiellement inséré dans l'ouverture ou l'orifice au niveau de l'extrémité distale de la tige allongée (110), et l'effecteur terminal (120, 125) étant fixé dans l'ouverture ou l'orifice par sertissage.

13. Sonde d'instrument chirurgical selon la revendication 1, dans laquelle l'isolation (114) est une isolation themorétractable.

14. Sonde d'instrument chirurgical selon la revendication 1, dans laquelle l'isolation (114) s'étend sur une surface externe de la partie distale du moyeu de poignée de crayon (130).
